# EUROPEAN PATENT APPLICATION

(11) **EP 1 050 758 A1**
(43) Date of publication of application: **08.11.2000**
(21) Application number: 99108723.0
(22) Date of filing: 03.05.1999
(51) Int. Cl.: G01N 33/68, A61K 38/18

(54) **Methods of diagnosing or treating neuropsychiatric diseases on basis of increased cerebrospinal fluid levels of neurotrophin 3**

(71) Applicant: Evotec BioSystems AG, 22525 Hamburg (DE)
(72) Inventor: The designation of the inventor has not yet been filed
(74) Representative: Meyers, Hans-Wilhelm, Dr.Dipl.-Chem.

(57) **Abstract**

The invention relates to a method for diagnosing or prognosing a neurodegenerative or neuropsychiatric disease in a subject, or determining whether a subject is at increased risk of developing a neurodegenerative or neuropsychiatric disease, comprising:
determining a level of neurotrophin 3 and/or of a transcription product of a gene coding for neurotrophin 3 in a sample from said subject;
and comparing said level to a reference value representing a known disease or health status,
thereby diagnosing or prognosing said neurodegenerative or neuropsychiatric disease in said subject, or determining whether said subject is at increased risk of developing a neurodegenerative or neuropsychiatric disease.

## Description

Alzheimer's disease (AD), first described by the Bavarian psychiatrist Alois Alzheimer in 1907, is a progressive neuropsychiatric disorder which begins with short term memory loss and proceeds to loss of cognitive functions, disorientation, impairment of judgement and reasoning and, ultimately, dementia. It is the most common form of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2,* as well as to mutations of the amyloid precursor gene *APP.* The majority of AD patients have no obvious family history and are classified as sporadic AD. For this late onset AD, several putative genetic risk factors have been reported. Among these the ApoE-epsilon 4 allele (ApoE4) has been widely confirmed to confer increased risk of developing AD. Inheritance of ApoE4 and other risk factors are neither necessary nor sufficient to cause AD. In contrast to the APP- and PSEN mutations which increase the production of Aβ, the principal component of senile plaques in AD brain, the ApoE variant most likely influences Aβ accumulation by modulating clearance and degradation of the peptide. As AD is a growing social and medical problem, there is a strong need for methods of diagnosing or prognosing said disease in subjects as well as for methods of treatment.

Despite its relevance for quality care initiatives, the field of psychiatry has little scientific knowledge regarding the course of current major depression when primary care patients with the disorder remain undetected. The risk that undetected patients develop suicidal ideation should not be underestimated. Therefore, methods of diagnosing major depression as well as methods of treatment are urgently needed.

In the search for biochemical changes in patients with neuropsychiatric disorders analysis of cerebrospinal fluid (CSF) may be a useful method, since the CSF is continuous with the extracellular fluid of the brain. Therefore, a plurality of studies aiming at the analysis of the central nervous system (CNS) specific proteins in CSF were performed in order to find biochemical markers for neuronal and synaptic function and pathology in degenerative brain disorders.

Neurotrophin 3 (NT-3) is a member of the neurotrophin gene family which supports the survival, differentiation, maintenance, and repair of vertebrate neurons (Bothwell, Ann. Rev. Neurosci. 8, 223 - 253, 1995). It was shown to prevent the death of adult central noradrenergic neurons of the locus coeruleus in a 6-hydorxydopamine lesion model (Arenas et. al., Nature 367(6461), 368 - 371, 1994), a neuronal population which is associated with the pathophysiology of major depression (Leonard, J. Psychopharmacol., 11(4) 39 - 47, 1997). Alterations in adrenoreceptor density and function, and changes in adrenoreceptors associated with the pituitary-adrenal axis function strongly implicate a disorder in central noradrenergic transmission in major depression (for review see: Leonard, J. Psychopharma 11(4), 39 - 47, 1997). This dysfunction might be related to the activity of tyrosine hydroxylase, the rate-limiting enzyme in the synthesis of catecholamines. Alternatively, impaired uptake or transport of a target-derived neurotrophic factor, such as NT-3, may contribute to central noradrenergic dysfunction.

Up to date, studies on CSF levels of NT-3 in major depression have not yet been reported. NT-3 was previously determined in the CSF of patients with a variety of neuropsychiatric conditions including hydrocephalus, meningitis, encephalitis, ventriculitis, brain tumors, and multiple sclerosis, with values ranging form 4.8 to 55.9 pg/ml (Gilmore et al., Psychiatry Res. 73(1-2) 109- 113, 1997). In the report of Gilmore, NT-3 was not detectable in the CSF of patients with schizophrenia.

Interestingly, Smith et al. (Proc. Natl. Acad. Sci. USA, 92(19), 8788 - 8792, 1995) showed that chronic treatment with antidepressants that selectively blocked neuronal noradrenaline uptake decreased the expression levels of NT-3 in the locus coeruleus in rats. In contrast, treatment with selective serotonin reuptake inhibitors did not alter NT-3 mRNA levels. These findings suggest that some effects of antidepressants on the function of the locus coeruleus involve NT-3 expression. However, these findings make it highly unlikely that the increased CSF levels of NT-3 in patients with DE - which is the subject of the present invention in detail described below - are due to effects of treatment with antidepressants.

In one aspect, the invention features a method for diagnosing or prognosing a neurodegenerative or neuropsychiatric disease in a subject, or determining whether a subject is at increased risk of developing a neurodegenerative or neuropsychiatric disease, comprising:
determining a level of neurotrophin 3 and/or of a transcription product of a gene coding for neurotrophin 3 in a sample from said subject;
and comparing said level to a reference value representing a known disease or health status,
thereby diagnosing or prognosing said neurodegenerative or neuropsychiatric disease in said subject, or determining whether said subject is at increased risk of developing a neurodegenerative or neuropsychiatric disease.

It is preferred to use a body fluid, preferably cerebrospinal fluid, as said sample. An increase of a level of neurotrophin 3 in said cerebrospinal fluid from said subject relative to said reference value representing a known health status indicates a diagnosis, or prognosis, or increased risk of Alzheimer's disease and/or major depression in said subject. The occurence of one or two ApoE4 alleles in the subject's cells further indicates a diagnosis of, or prognosis of, or increased risk of developing, Alzheimer's disease.

In a further aspect, the invention features a method of monitoring progression of a neurodegenerative or neuropsychiatric disease, in particular Alzheimer's disease and/or major depression, in a subject, comprising:
determining a level of neurotrophin 3 and/or of a transcription product of a gene coding for neurotrophin 3 in a sample from said subject;
and comparing said level to a reference value representing a known disease or health status, thereby monitoring progression of said neurodegenerative or neuropsychiatric disease in said subject.

It is particularly preferred to compare a level of neurotrophin 3 and/or of a transcription product of a gene coding for neurotrophin 3 in said sample with a level of at least one of said substances in a series of samples taken from said subject over a period of time.

In still a further aspect, the inventions features a method of evaluating a treatment for a neurodegenerative or neuropsychiatric disease, in particular Alzheimer's disease and/or major depression, comprising:
determining a level of neurotrophin 3 and/or of a transcription product of a gene coding for neurotrophin 3 in a sample from said subject;
and comparing said level to a reference value representing a known disease or health status,
thereby evaluating said treatment for said neurodegenerative or neuropsychiatric disease.

In another aspect, the invention features a kit for diagnosis, or prognosis, or determination of increased risk of developing a neurodegenerative or neuropsychiatric disease, in particular Alzheimer's disease or major depression, in a subject, said kit comprising:
(a) at least one reagent which selectively detects neurotrophin 3 or a transcription product of a gene coding for neurotrophin 3; and
(b) instructions for diagnosing, or prognosing said neurodegenerative or neuropsychiatric disease, or determining increased risk of developing said neurodegenerative or neuropsychiatric disease by
   (i) detecting a level of neurotrophin 3 and/or of a transcription product of a gene coding for neurotrophin 3 in a sample from said subject; and
   (ii) diagnosing, or prognosing, or determining whether said subject is at increased risk of developing said neurodegenerative or neuropsychiatric disease,
wherein an increased level of neurotrophin 3 and/or of a transcription product of a gene coding for neurotrophin 3 compared to a reference value representing a known health status;
or a level of neurotrophin 3 and/or of a transcription product of a gene coding for neurotrophin 3 similar or equal to a reference value representing a known disease status indicates a diagnosis, or prognosis, or increased risk of developing said neurodegenerative or neuropsychiatric disease.

In still another aspect, the invention features a method of treating or preventing a neurodegenerative or neuropsychiatric disease, in particular Alzheimer's disease and/or major depression, in a subject comprising administering to said subject in a therapeutically effective amount an agent or agents which directly or indirectly reduce an activity, or level, or both said activity and level, of at least one substance which is selected from the group consisting of a gene coding for neurotrophin 3, its non-coding regulatory elements, a transcription product of a gene coding for neurotrophin 3, and neurotrophin 3.

In still another aspect, the invention features a use of an agent for treating a neurodegenerative or neuropsychiatric disease, in particular Alzheimer's disease and/or major depression, wherein said agent directly or indirectly reduces an activity, or level, or both said activity and level, of at least one substance which is selected from the group consisting of a gene coding for neurotrophin 3, its non-coding regulatory elements, a transcription product of a gene coding for neurotrophin 3, and neurotrophin 3.

In still another aspect, the invention features a method for identifying an agent that directly or indirectly affects an activity, or level, or both said activity and level, of at least one substance which is selected from the group consisting of a gene coding for neurotrophin 3, its non-coding regulatory elements, a transcription product of a gene coding for neurotrophin 3, and neurotrophin 3, comprising the steps of:
(a) providing a sample containing at least one substance which is selected from the group consisting of a gene coding for neurotrophin 3, its non-coding regulatory elements, a transcription product of a gene coding for neurotrophin 3, and neurotrophin 3;
(b) contacting said sample with at least one agent;
(c) comparing an activity, or level, or both said activity and level, of at least one of said substances before and after said contacting.

The increases in CSF levels of NT-3 may reflect disturbances in the trophic support of specific neuronal populations, such as the basal forebrain cholinergic system in AD and the central noradrenergic system in DE.

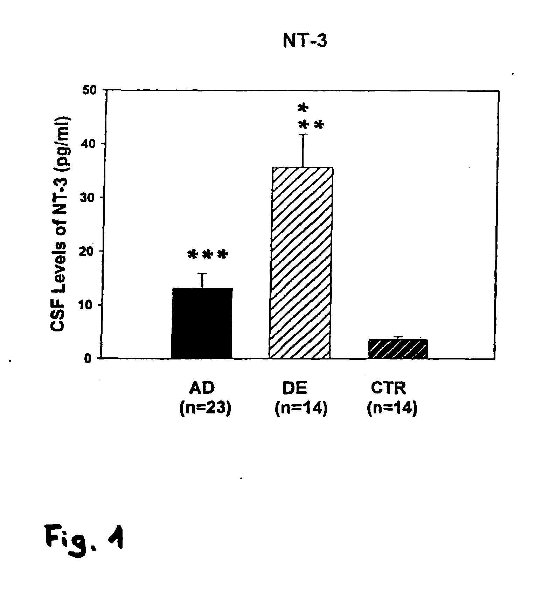

Figure 1 depicts that CSF levels of NT-3 were significantly elevated in the DE group, as compared to both the AD and the CTR groups. Levels (pg/ml) are given in mean ± SEM. Asterisk (*, **, ***) indicate significance (p < 0.005), Mann-Whitney U Test. NT-3: * DE versus AD, p = 0.004; ** DE versus CTR, p < 0.001; *** AD versus CTR, p = 0.013. NT-3 concentrations in CSF of the DE group were 35.7 +/- 6.2 pg/ml (mean +/- SEM; range 2.3 to 87.00, n = 14), compared to 13.2 +/- 2.8 pg/ml in the AD group (range: 2.0 to 41.0, n = 23), and 3.7 +/- 0.5 pg/ml in the CTR group (range: 0.00 to 8.2, n = 14), respectively. In addition, CSF levels of NT-3 were significantly higher in the AD group as compared to the CTR group. There was no apparent correlation of CSF levels of NT-3 with age, duration of AD, MMS, NOSGER or MADRS scores.

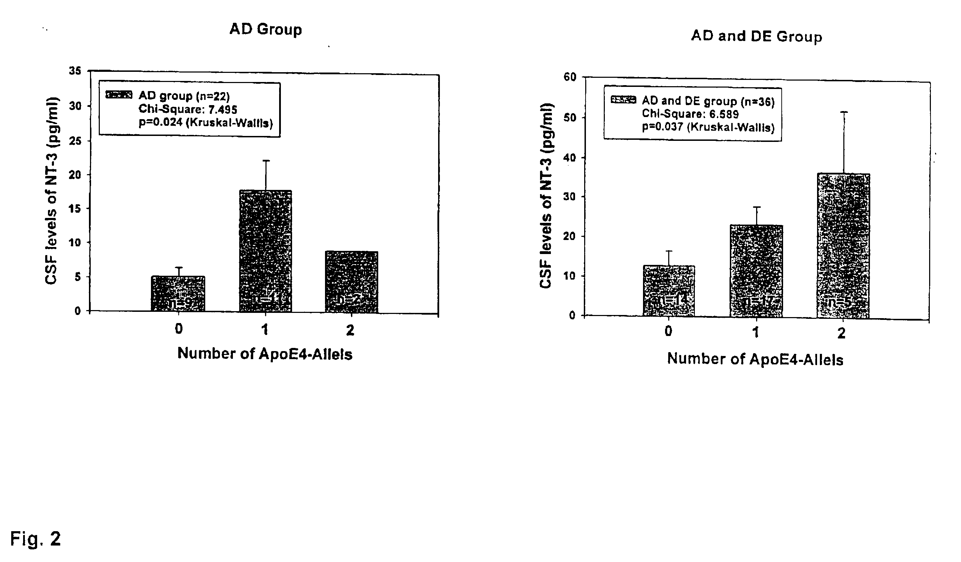

Figure 2 depicts CSF levels of NT-3 (pg/ml ± SEM) grouped for presence of ApoE4 alleles. Left panel: Alzheimer's disease group, n = 22, Kruskal-Wallis: χ² = 7.495, p = 0.024. Right panel: total sample of patients with AD and major depression (DE), n = 36, Kruskal-Wallis test: χ² = 6.589, p = 0.037. CSF levels of NT-3 increased with increasing ApoE4 allele frequency in the total sample of AD and DE patients and within the AD group. There was no apparent effect of the number of E4 alleles within the DE group (n = 14, Kruskal-Wallis test: χ² = 1.508, p = 0.471).

### EXAMPLE 1

In order to achieve a differential diagnosis, the study included not only patients with AD, but also such with major depression (DE). Diagnosis of probable AD was made according to criteria of the National Institute of Neuropsychiatric and Communicative Disorders and Stroke-Alzheimer's disease and Related Disorders Association (NINCDS-ADRDA; McKhann et al., Neurology 34, 939 - 944, 1984). Patients with major depression were diagnosed according to the ICD10 (F32.0x/1x, F33.0x/1x) and DSM-IIIR (296.20-22, 296.30-32) criteria. All patients were referred to the research ward from general practitioners, neurologists and psychiatrists for diagnostic purposes and screening for clinical trials. None of the patients was institutionalized. The group of healthy control subjects (CTR) consisted of patients that underwent lumbar puncture for orthopedic or neurologic diagnostic purposes and were shown to have normal CSF cell counts, total protein levels, and absence of signs of blood barrier dysfunction or cerebral IgG synthesis, as well as absence of any cerebral disorders.

AD, DE and CTR patients were carefully examined and received a thorough clinical work-up. Psychometric testing including the Mini Mental State (MMS; Folstein et al., J. Psychiatry Res. 12, 189- 198, 1975), as a global screening instrument for dementia, and the Nurses' Observation Scale for Geriatric Patients (NOSGER; Spiegel et al., J. Am. Geriatr. Soc. 39(4), 339 - 347, 1991) as a functional measure of dementia severity. The patients with DE showed no cognitive disturbances in the clinical examinations and the Mini Mental State scores were within the normal range. Severity of depression was rated by using the Montgomery Asberg Depression Rating Scale (MADRS) (Montgomery et al., Br. J. Psychiatry, 134: 382 - 389, 1979). Apolipoprotein (ApoE) genotyping, or, if DNA was not available, ApoE phenotyping was included in the laboratory screening in the AD patients.

CSF was obtained for diagnostic purposes in the AD and DE patients in which no lumbar puncture had been previously done during the routine diagnostic work-up. Different CSF volumes were available for the analysis of the neurotrophin proteins. This fact explains the different sample sizes for the individual measurements. All available CSF samples were used for the analyses.

The AD group was as follows: n = 23, 12 men, 11 women, mean age 63.9 +/- 13.2 SD years, range 39 - 86 years, MMS score: mean 18.6 +/- 5.6 SD.

The DE group was as follows: n = 14, 5 men, 9 women, mean age 68.2 +/- 13.6 SD years, range 47 - 86 years, MMS score: mean 28.1 +/- 0.9 SD.

The CTR group was as follows: n = 14, 8 men, 6 women, mean age 58.5 +/- 16.2 SD years, range 31 - 81 years.

AD and CTR patients were free of psychotropic medication. Patients with major depression were treated with various antidepressant drugs including serotonin reuptake inhibitors, reversible monoaminooxidase A inhibitors and tricyclics. Informed consent was taken from each patient and their caregivers before the investigation. The study was approved by the local ethics committee. All procedures were in accordance with the Helsinki Declaration of 1975, as revised in 1983. Within one week of dementia testing, CSF was obtained by lumbar puncture. To control for possible influences of a ventriculo-lumbar gradient, lumbar punctures were done between 7.30 and 8 a. m. before breakfast while patients were still lying flat. CSF samples were frozen on dry ice immediately upon withdrawal at the bedside in 0.5 ml aliquots and stored at - 85 °C until biochemical analysis.

NT-3 was determined using commercially available ELISA systems (Promega, Madison, WI). The determination was performed according to the manufacture's protocol. 220 µl of undiluted CSF in carbonate buffer (pH 9.7) were added to 96 well immunoplates (Nunc) at 4 °C overnight. Anti-Human-NT-3 polyclonal antibodies (pAb) was used as capture antibody. Anti-NT-3 mAb was used as reporter antibody. After incubation with a species-specific Ab (anti-rat IgG) conjugated to horseradish peroxidase (HRP) as a tertiary reactant, and washing, the solution was incubated with the chromogenic substrate TMB (3, 5, 3', 5'-tetramethylbenzidine). Absorbance was measured at 450 nm using a microplate reader (Dynatech MR 700). NT-3 ELISA: range 4.7 - 300 pg/ml; cross-reaction with other neurotrophins at 10 ng/ml < 3 %; detection limit 10.0 pg/ml.

ApoE genotyping was performed using INNO-LiPA ApoE, Innogenetics, Belgium. ApoE phenotyping was performed according to McDowell et al. (Clin. Chem. 35(10), 2070 - 2073, 1989). The use of the ApoE phenotype synonymous with the ApoE genotype in the statistical analyses seemed to be appropriate, since ApoE genotyping compared with protein phenotyping showed conflicting results in less than 2 % (Hansen et al., Clin. Chim. Acta, 224(2), 131 - 137, 1994).

Statistical analyses of data were performed using the Mann-Whitney U test for group comparisons. Correlation analyses were performed by multiple regression using CSF levels of neurotrophins as well as ApoE genotype (or phenotype, respectively), age, duration of the disease in AD, MMS, NOSGER and MADRS scores. Non-parametric correlation was performed using the Kruskal-Wallis test. Regression analysis was complemented with analysis of variance (ANOVA) by using SPSS for Windows (version 8.0). Statistical significance was assumed at p < 0.05. Bonferroni correction for multiple testing was applied.

## Claims

1. A method for diagnosing or prognosing a neurodegenerative or neuropsychiatric disease in a subject, or determining whether a subject is at increased risk of developing a neurodegenerative or neuropsychiatric disease, comprising:
determining a level of neurotrophin 3 and/or of a transcription product of a gene coding for neurotrophin 3 in a sample from said subject;
and comparing said level to a reference value representing a known disease or health status,
thereby diagnosing or prognosing said neurodegenerative or neuropsychiatric disease in said subject, or determining whether said subject is at increased risk of developing said neurodegenerative or neuropsychiatric disease.

2. The method according to claim 1, wherein said sample is a body fluid, preferably cerebrospinal fluid.

3. The method according to claim 2, wherein an increase of said level of neurotrophin 3 in said cerebrospinal fluid from said subject relative to said reference value representing a known health status indicates a diagnosis, or prognosis, or increased risk of Alzheimer's disease and/or major depression in said subject.

4. The method according to claim 3, wherein the occurence of one or two ApoE4 alleles in the subject's cells indicates a diagnosis of, or prognosis of, or increased risk of developing, Alzheimer's disease.

5. A method of monitoring progression of a neurodegenerative or neuropsychiatric disease, in particular Alzheimer's disease and/or major depression, in a subject, comprising:
determining a level of neurotrophin 3 and/or of a transcription product of a gene coding for neurotrophin 3 in a sample from said subject;
and comparing said level to a reference value representing a known disease or health status, thereby monitoring progression of said neurodegenerative or neuropsychiatric disease in said subject.

6. A method according to claim 5, further comprising comparing a level of neurotrophin 3 and/or of a transcription product of a gene coding for neurotrophin 3 in said sample with a level of at least one of said substances in a series of samples taken from said subject over a period of time.

7. A method of evaluating a treatment for a neurodegenerative or neuropsychiatric disease, in particular Alzheimer's disease and/or major depression, comprising:
determining a level of neurotrophin 3 and/or of a transcription product of a gene coding for neurotrophin 3 in a sample from said subject;
and comparing said level to a reference value representing a known disease or health status,
thereby evaluating said treatment for said neurodegenerative or neuropsychiatric disease.

8. A kit for diagnosis, or prognosis, or determination of increased risk of developing a neurodegenerative or neuropsychiatric disease, in particular Alzheimer's disease or major depression, in a subject, said kit comprising:
(a) at least one reagent which selectively detects neurotrophin 3 or a transcription product of a gene coding for neurotrophin 3; and
(b) instructions for diagnosing, or prognosing said neurodegenerative or neuropsychiatric disease, or determining increased risk of developing said neurodegenerative or neuropsychiatric disease by
(i) detecting a level of neurotrophin 3 and/or of a transcription product of a gene coding for neurotrophin 3 in a sample from said subject; and
(ii) diagnosing, or prognosing, or determining whether said subject is at increased risk of developing said neurodegenerative or neuropsychiatric disease,
wherein an increased level of neurotrophin 3 and/or of a transcription product of a gene coding for neurotrophin 3 compared to a reference value representing a known health status;
or a level of neurotrophin 3 and/or of a transcription product of a gene coding for neurotrophin 3 similar or equal to a reference value representing a known disease status
indicates a diagnosis, or prognosis, or increased risk of developing said neurodegenerative or neuropsychiatric disease.

9. A method of treating or preventing a neurodegenerative or neuropsychiatric disease, in particular Alzheimer's disease and/or major depression, in a subject comprising administering to said subject in a therapeutically effective amount an agent or agents which directly or indirectly reduce an activity, or level, or both said activity and level, of at least one substance which is selected from the group consisting of a gene coding for neurotrophin 3, its non-coding regulatory elements, a transcription product of a gene coding for neurotrophin 3, and neurotrophin 3.

10. Use of an agent for treating a neurodegenerative or neuropsychiatric disease, in particular Alzheimer's disease and/or major depression, wherein said agent directly or indirectly reduces an activity, or level, or both said activity and level, of at least one substance which is selected from the group consisting of a gene coding for neurotrophin 3, its non-coding regulatory elements, a transcription product of a gene coding for neurotrophin 3, and neurotrophin 3.

11. A method for identifying an agent that directly or indirectly affects an activity, or level, or both said activity and level, of at least one substance which is selected from the group consisting of a gene coding for neurotrophin 3, its non-coding regulatory elements, a transcription product of a gene coding for neurotrophin 3, and neurotrophin 3, comprising the steps of:
(a) providing a sample containing at least one substance which is selected from the group consisting of a gene coding for neurotrophin 3, its non-coding regulatory elements, a transcription product of a gene coding for neurotrophin 3, and neurotrophin 3;
(b) contacting said sample with at least one agent;
(c) comparing an activity, or level, or both said activity and level, of at least one of said substances before and after said contacting.
